(19) Europäisches Patentamt
European Patent Office
Office européen des brevets

(11) Veröffentlichungsnummer: **0 334 146**
**A1**

(12) # EUROPÄISCHE PATENTANMELDUNG

(21) Anmeldenummer: 89104376.2

(22) Anmeldetag: 13.03.89

(51) Int. Cl.⁴: C07D 413/04 , A61K 31/535 , //C07D213/61

(30) Priorität: 23.03.88 DE 3809775

(43) Veröffentlichungstag der Anmeldung: 27.09.89 Patentblatt 89/39

(84) Benannte Vertragsstaaten: AT BE CH DE ES FR GB GR IT LI LU NL SE

(71) Anmelder: Dr. Karl Thomae GmbH
Postfach 1755
D-7950 Biberach 1(DE)

(72) Erfinder: Reiffen, Manfred, Dr. Dipl.-Chem.
Matthias-Erzberger-Strasse 40
D-7950 Biberach 1(DE)
Erfinder: Mark, Michael, Dr.
Schlehenhang 17
D-7950 Biberach 1(DE)
Erfinder: Sauter, Robert, Dr. Dipl.-Chem.
Albert-Schweitzer-Weg 9
D-7958 Laupheim(DE)
Erfinder: Grell, Wolfgang, Dr. Dipl.-Chem.
Geschwister-Scholl-Strasse 18
D-7950 Biberach 1(DE)

(54) **Neue Morpholine und Morpholin-N-oxide, diese Verbindungen enthaltende Arzneimittel und Verfahren zu ihrer Herstellung.**

(57) Die Erfindung betrifft neue Morpholine und Morpholin-N-oxide der Formel

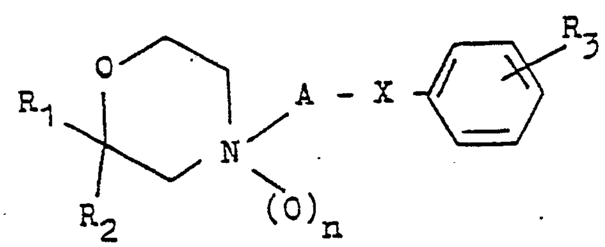

,(I)

in der
A eine gegebenenfalls durch Methyl- oder Ethylgruppen mono-oder disubstituierte n-Alkylengruppe,
X eine Bindung oder ein Sauerstoffatom,
$R_1$ eine gegebenenfalls durch ein Halogenatom, eine Trifluormethyl- oder Alkylgruppe substituierten heteroaromatischen 6-gliedrigen Ring,
$R_2$ ein Wasserstoffatom oder eine Hydroxygruppe,
$R_3$ eine Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe, die endständig durch eine Hydroxy-, Alkoxy-, Phenylalkoxy-, Carboxy-, Alkoxycarbonyl-, Aminocarbonyl-, Alkylaminocarbonyl- oder Dialkylaminocarbonylgruppe substituiert ist, oder eine gegebenenfalls durch eine Alkylgruppe substituierte Ethenylengruppe, die endständig durch eine Carboxy-, Alkoxycarbonyl-, Aminocarbonyl-, Alkylaminocarbonyl-, Dialkylaminocarbonyl-, Pyrrolidino-, Piperidino- oder Hexamethyleniminogruppe substituiert ist, und
n die Zahl 0 oder 1, bedeuten, deren optische Isomere, Diastereomere und deren Säureadditionssalze.
Die neuen Verbindungen der obigen Formel I, deren optische Isomere und deren Diastereomere sowie

EP 0 334 146 A1

deren Säureadditionssalze, insbesondere deren physiologisch verträgliche Säureadditionssalze mit anorganischen oder organischen Säuren, weisen wertvolle pharmakologische Eigenschaften auf, nämlich eine Wirkung auf den Stoffwechsel, vorzugsweise eine blutzuckersenkende und körperfettreduzierende Wirkung, sowie eine senkende Wirkung auf die atherogenen ß-Lipoproteine VLDL und LDL. Außerdem weisen diese eine leistungssteigernde Wirkung bei Tieren auf.

Die neuen Verbindungen der obigen Formel I lassen sich nach an und für sich bekannten Verfahren herstellen.

## Neue Morpholine und Morpholin-N-oxide, diese Verbindungen enthaltende Arzneimittel und Verfahren zu ihrer Herstellung

Gegenstand der vorliegenden Erfindung sind neue Morpholine und Morpholin-N-oxide der Formel

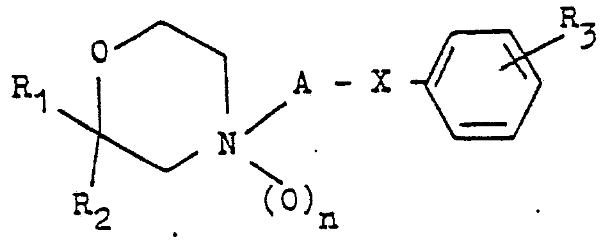

,(I)

deren optische Isomere und Diastereomere, da die neuen Verbindungen ein oder mehrere optisch aktive Kohlenstoffatome enthalten, sowie deren Säureadditionssalze, insbesondere für die pharmazeutische Anwendung deren physiologisch verträglichen Säureadditionssalze mit anorganischen oder organischen Säuren, welche wertvolle pharmakologische Eigenschaften aufweisen, nämlich eine Wirkung auf den Stoffwechsel, vorzugsweise eine blutzuckersenkende und körperfettreduzierende Wirkung, sowie eine senkende Wirkung auf die atherogenen Lipoproteine VLDL und LDL. Außerdem weisen einige der vorstehend erwähnten Verbindungen auch eine anabole Wirkung auf.

In der obigen Formel bedeutet
A eine gegebenenfalls durch Methyl- oder Ethylgruppen mono-oder disubstituierte n-Alkylengruppe mit 2 oder 3 Kohlenstoffatomen,
X eine Bindung oder ein Sauerstoffatom,
$R_1$ eine gegebenenfalls durch ein Halogenatom, eine Trifluormethyl- oder Alkylgruppe substituierten heteroaromatischen 6-gliedrigen Ring, der 1 oder 2 Stickstoffatome enthält,
$R_2$ ein Wasserstoffatom oder eine Hydroxygruppe,
$R_3$ eine Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe mit 1 bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, eine Alkoxygruppe mit 2 bis 7 Kohlenstoffatomen, die endständig durch eine Hydroxy-, Alkoxy- oder Phenylalkoxygruppe substituiert ist, oder eine gegebenenfalls durch eine Alkylgruppe substituierte Ethenylengruppe, die endständig durch eine Carboxy-, Alkoxycarbonyl-, Aminocarbonyl-, Alkylaminocarbonyl-, Dialkylaminocarbonyl-, Pyrrolidino-, Piperidino- oder Hexamethyleniminogruppe substituiert ist, und
n die Zahl 0 oder 1,
wobei alle vorstehend erwähnten Alkyl- oder Alkoxygruppen jeweils 1 bis 3 Kohlenstoffatome enthalten können.

Für die bei der Definition der Reste eingangs erwähnten Bedeutungen kommt beispielsweise für A die der Ethylen-, 1-Methyl-ethylen-, 2-Methyl-ethylen-, 1-Ethyl-ethylen-, 2-Ethylen-ethylen-, 1,2-Dimethyl-ethylen-, 1,1-Dimethyl-ethylen-, 1,1-Diethyl-ethylen-, 1-Ethyl-1-methyl-ethylen-, 2,2-Dimethyl-ethylen-, 2,2-Diethyl-ethylen-, 2-Ethyl-2-methyl-ethylen-, n-Propylen-, 1-Methyl-n-propylen-, 2-Methyl-n-propylen-, 3-Methyl-n-propylen-, 1-Ethyl-n-propylen-, 2-Ethyl-n-propylen-, 3-Ethyl-n- propylen, 1,1-Dimethyl-n-propylen-, 1,1-Diethyl-n-propylen-, 2,3-Dimethyl-n-propylen-, 3,3-Dimethyl-n-propylen- oder 3-Ethyl-3-methyl-n-propylengruppe,
für $R_1$ die der Pyridin-2-yl-, Pyridin-3-yl-, Pyridin-4-yl-, Pyridazin-3-yl-, Pyridazin-4-yl-, Pyrimidin-2-yl-, Pyrimidin-4-yl-, Pyrimidin-5-yl-, Pyrazin-2-yl-, 2-Chlor-pyridin-6-yl-, 2-Chlor-pyridin-4-yl-, 2-Brom-pyridin-4-yl-, 2-Brom-pyridin-6-yl-, 3-Chlor-pyridin-5-yl-, 3-Brom-pyridin-5-yl-, 2-Trifluormethyl-pyridin-4-yl-, 2-Trifluormethyl-pyridin-6-yl-, 2-Methyl-pyridin-4-yl-, 2-Ethyl-pyridin-4-yl-, 2-Propyl-pyridin-4-yl-, 2-Isopropyl-pyridin-4-yl-, 3-Methyl-pyridin-2-yl-, 4-Methyl-pyridin-2-yl-, 2-Methyl-pyridin-3-yl-, 4-Methyl-pyridin-3-yl oder 3-Methyl-pyridin-5-yl-gruppe,
für $R_2$ die des Wasserstoffatoms oder die der Hydroxygruppe,
für X die des Sauerstoffatoms oder einer Bindung und
für $R_3$ die der Hydroxy-, Methoxy-, Ethoxy-, n-Propoxy-, Isopropoxy-, Carboxy-, Methoxycarbonyl-, Ethoxycarbonyl-, n-Propoxycarbonyl-, Aminocarbonyl-, Methylaminocarbonyl-, Ethylaminocarbonyl-, Isopropylaminocarbonyl-, Dimethylaminocarbonyl-, Diethylaminocarbonyl-, Di-n-propylaminocarbonyl-, N-Ethyl-methylaminocarbonyl-, N-Ethyl-isopropylaminocarbonyl-, 2-Hydroxy-ethoxy-, 3-Hydroxy-n-propoxy-, 4-Hydroxy-n-butoxy-, 5-Hydroxy-n-pentoxy-, 6-Hydroxy-n-hexoxy-, 7-Hydroxy-n-heptoxy-, 2-Methoxy-ethoxy-, 2-Ethoxy-ethoxy-, 2-n-Propoxy-ethoxy-, 3-Ethoxy-n-propoxy-, 4-Methoxy-n-butoxy-, 6-Ethoxy-n-hexoxy-, 2-Phenethoxy-ethoxy-, Carboxymethoxy-, 2-Carboxy-ethoxy-, 3-Carboxy-n-propoxy-, 4-Carboxy-n-butoxy-, Methoxycarbonylmethoxy-, 2-Methoxycarbonyl-ethoxy-, 6-Methoxycarbonyl-hexoxy-, Ethoxycarbonylmethoxy-, 2-Ethoxycarbonyl-ethoxy-, 3-Ethoxycarbonyl-n-propoxy-, n-Propoxycarbonylmethoxy-, 2-Isopropoxycarbonyl-ethoxy-, 4-n-Propoxycar bonyl-n-butoxy-, Aminocarbonylmethoxy-, 2-Aminocarbonyl-ethoxy-, 4-Aminocarbonyl-n-butoxy-, Methylaminocarbonylmethoxy-, 2-Methylaminocarbonyl-ethoxy-, Dimethylaminocarbonyl-methoxy-, 2-Dimethylaminocarbonyl-ethoxy-, 4-Dimethylaminocarbonyl-n-butoxy-, Diethylaminocarbonyl-methoxy-, 2-Diethylaminocarbonyl-ethoxy-, 2-Di-n-propyl-aminocarbonyl-ethoxy-, 2-Carboxy-ethenyl-, 2-Carboxy-1-methyl-ethenyl-, 2-Carboxy-2-methyl-ethenyl-, 2-Carboxy-1-ethyl-ethenyl-, 2-Carboxy-1-n-propyl-ethenyl-, 2-Methoxycarbonyl-ethenyl-, 2-Methoxycarbonyl-1-methyl-ethenyl-, 2-Ethoxycarbonyl-ethenyl-, 2-Ethoxycarbonyl-1-methyl-ethenyl-, 2-Isopropoxycarbonyl-ethenyl-, 2-Pyrrolidino-ethoxy-, 2-Piperidino-ethoxy-oder 2-Hexamethylenimino-ethoxygruppe in Betracht.

Beispielsweise seien zusätzlich zu den in den Beispielen genannten Verbindungen noch folgende Verbindungen genannt, die unter die vorstehend erwähnte allgemeine Formel I fallen:

N-[2-(4-Carboxymethoxyphenyl)-1-methylethyl]-2-(6-trifluormethyl-pyridin-2-yl)-morpholin,
N-[2-(4-Carbomethoxymethoxyphenyl)-1-methylethyl]-2-(6-trifluormethyl-pyridin-2-yl)-morpholin,
N-[2-(4-(2-Hydroxy-ethoxy)-phenyl)-1-methylethyl]-2-(6-trifluormethyl-pyridin-2-yl)-morpholin,
N-[2-(4-Carbomethoxymethoxyphenyl)-1-methylethyl]-2-hydroxy-2-(6-chlor-pyridin-2-yl)-morpholin,
N-[2-(4-Carboxymethoxyphenyl)-1-methylethyl]-2-hydroxy-2-(6-chlor-pyridin-2-yl)-morpholin,
N-[2-(4-(2-Hydroxy-ethoxy)-phenyl)-1-methylethyl]-2-hydroxy-2-(6-chlor-pyridin-2-yl)-morpholin,
N-[2-(4-(2-Carbomethoxy-1-methylethenyl)-phenyl)-1-methylethyl]-2-(6-methyl-pyridin-2-yl)-morpholin,
N-[2-(4-Carbomethoxymethoxy-phenoxy)-ethyl]-2-(6-methyl-pyridin-2-yl)-morpholin,
N-[2-(4-(2-(1-Piperidino)-ethoxy)-phenyl)-1-methylethyl]-2-(6-chlor-pyridin-2-yl)-morpholin,
N-[2-(4-Aminocarbonylmethoxy-phenyl)-1-methylethyl]-2-(6-chlor-pyridin-2-yl)-morpholin,
N-[2-(4-Methylaminocarbonylmethoxy-phenyl)-1-methylethyl]-2-(6-chlor-pyridin-2-yl)-morpholin,
N-[2-(4-(2-Ethoxy-ethoxy)-phenyl)-1-methylethyl]-2-(6-chlor-pyridin-2-yl)-morpholin,
N-[2-(4-Carboxymethoxy-phenyl)-1-methylethyl]-2-(6-chlor-pyridin-2-yl)-morpholin,
N-[2-(4-Carboxy-phenyl)-1-methylethyl]-2-(6-chlor-pyridin-2-yl)-morpholin,
N-[2-(4-(2-(2-Phenylethoxy)-ethoxy)-phenyl)-1-methylethyl]-2-(6-chlor-pyridin-2-yl)-morpholin und
N-[2-(4-(6-Hydroxy-n-hexoxy)-phenyl)-1-methylethyl]-2-(6-chlor-pyridin-2-yl)-morpholin,
deren optisch Isomere, deren Diastereomere und deren Säureadditionssalze.

Bevorzugt sind jedoch die Verbindungen der Formel I, in der
n wie eingangs definiert ist,
A eine gegebenenfalls durch eine Methylgruppe substituierte Ethylen- oder n-Propylengruppe,
X eine Bindung,
$R_1$ einen gegebenenfalls durch ein Chlor- oder Bromatom substituierten Pyridinylrest, einen Pyrazinyl-, Pyrimidinyl-oder Pyridazinylrest,
$R_2$ ein Wasserstoffatom oder eine Hydroxygruppe und
$R_3$ eine Carboxy-, Methoxycarbonyl-, Ethoxycarbonyl-, Aminocarbonyl-, Carboxymethoxy-, Methoxycarbonylmethoxy-, Ethoxycarbonylmethoxy-, Aminocarbonylmethoxy-, Methylaminocarbonylmethoxy-, 2-Hydroxy-ethoxy-, 2-Ethoxy-ethoxy-, 2-Phenethoxy-ethoxy-, 6-Hydroxy-n-hexoxy- oder 2-Carbomethoxy-1-methyl-ethenylgruppe bedeuten, deren optisch Isomere, deren Diastereomere und deren Säureadditionssalze.

Besonders bevorzugt sind jedoch die Verbindungen der Formel

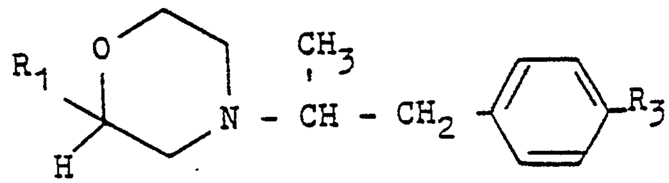

,(Ia)

in der
$R_1$ eine in 6-Stellung durch ein Chlor- oder Bromatom, durch eine Methyl- oder Trifluormethylgruppe

substituierte Pyridin-2-ylgruppe und
$R_3$ eine Carboxymethoxy-, Carbomethoxymethoxy-, Ethoxycarbonylmethoxy-, Aminocarbonylmethoxy-, Methylaminocarbonylmethoxy-, 2-Hydroxy-ethoxy-, 2-Ethoxy-ethoxy- oder 2-Carbomethoxy-1-methyl-ethenylgruppe bedeuten,
deren optische Isomere, deren Diastereomere und deren Säureadditionssalze.

Erfindungsgemäß erhält man die neuen Verbindungen nach folgenden Verfahren:

a) Zur Herstellung von Verbindungen der Formel I, in der n die Zahl 0 darstellt:
Umsetzung eines Ketons der Formel
$R_1$ - CO - $CH_2$ - $Z_1$ ,(II)
in der
$R_1$ wie eingangs definiert ist und
$Z_1$ eine nukleophile Austrittsgruppe darstellt, mit einem Ethanolamin der Formel

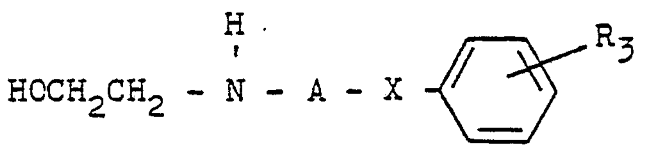
,(III)

in der
$R_3$, A und X wie eingangs definiert sind, und zur Herstellung einer Verbindung der Formel I, in der $R_2$ ein Wasserstoffatom darstellt, Reduktion einer so erhaltenen Verbindung der Formel

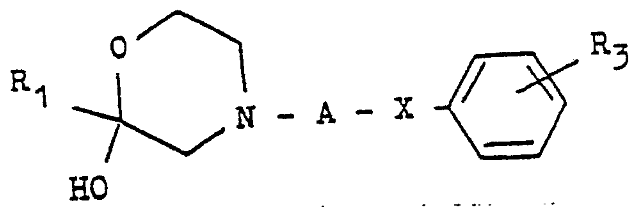
,(IV)

in der
$R_1$, $R_3$, A und X wie eingangs definiert sind, und gegebenenfalls anschließende Hydrolyse eines so erhaltenen Esters.

Als nukleophile Austrittsgruppen kommen beispielsweise Halogenatome oder Sulfonyloxygruppen, z.B. ein Chlor-, Brom-oder Jodatom, die Methansulfonyloxy-, p-Toluolsulfonyloxy-oder Ethoxysulfonyloxygruppe, in Betracht.

Die Umsetzung wird zweckmäßigerweise in einem geeigneten Lösungsmittel wie Aceton, Methylenchlorid, Tetrahydrofuran oder Dioxan und vorzugsweise in Gegenwart eines säurebindenden Mittels wie Kaliumhydrogencarbonat, Kaliumcarbonat, Natriumhydrogencarbonat, Natriumcarbonat, Triethylamin oder Pyridin, wobei die organischen säurebindenden Mittel gleichzeitig auch als Lösungsmittel verwendet werden können, bei Temperaturen zwischen 0 und 100 °C, vorzugsweise bei Temperaturen zwischen 5 und 50 °C, durchgeführt.

Die zur Herstellung einer Verbindung der Formel I, in der $R_2$ ein Wasserstoffatom und n die Zahl 0 darstellen, erforderliche anschließende Reduktion einer Verbindung der Formel IV wird vorzugsweise in einem Lösungsmittel wie Methylenchlorid, Chloroform, Trifluoressigsäure oder Trifluormethansulfonsäure mit einem geeigneten Hydrid wie einem komplexen Metallhydrid, z.B. mit Natriumborhydrid, mit katalytisch angeregtem Wasserstoff, z.B. mit Wasserstoff in Gegenwart von Platin, oder einem Trialkylsilan, z.B. mit Triethylsilan, gegebenenfalls in Gegenwart einer Säure wie Bortrifluorid, z.B. in Gegenwart von Bortrifluorid-Etherat, bei Temperaturen zwischen 0 und 60 °C, vorzugsweise jedoch in Trifluoressigsäure als Lösungsmittel und bei Raumtemperatur, durchgeführt.

Die gegebenenfalls anschließende Esterspaltung erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches.

b) Zur Herstellung von Verbindungen der Formel I, in der n die Zahl 0 und $R_2$ ein Wasserstoffatom

darstellen:
Reduktive Aminierung einer Carbonylverbindung der Formel

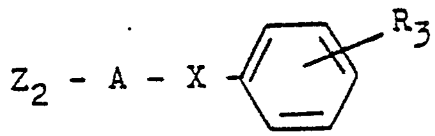

,(V)

in der
$R_3$, A und X wie eingangs definiert sind und
$Z_2$ zusammen mit einem Wasserstoffatom des benachbarten Kohlenstoffatoms des Restes A ein Sauerstoffatom darstellt, mit einem Amin der Formel

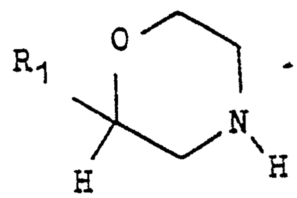

,(VI)

in der
$R_1$ wie eingangs definiert ist.

Die reduktive Aminierung wird in einem geeigneten Lösungsmittel wie Methanol, Ethanol, Tetrahydrofuran, Dioxan oder Acetonitril in Gegenwart eines geeigneten Reduktionsmittels wie einem geeigneten komplexen Metallhydrid, vorzugsweise jedoch in Gegenwart von Natriumcyanborhydrid bei einem pH-Wert von 5 bis 7, bei Temperaturen zwischen 0 und 50°C, vorzugsweise jedoch bei Raumtemperatur, durchgeführt.

c) Zur Herstellung von Verbindungen der allgemeinen Formel I, in der n die Zahl 0 und $R_2$ ein Wasserstoffatom bedeuten:
Reduktion einer Verbindung der allgemeinen Formel

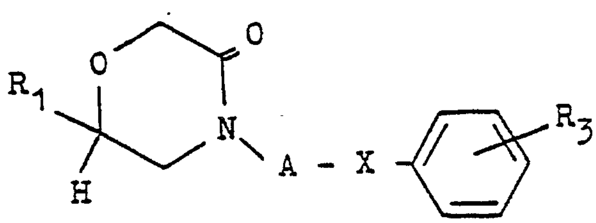

,(VII)

in der
A, X, $R_1$ und $R_3$ wie eingangs definiert sind.

Die Reduktion wird in einem geeigneten Lösungsmittel wie Diethylether oder Tetrahydrofuran mit einem Reduktionsmittel wie einem Metallhydrid, z.B. mit Lithiumaluminiumhydrid oder Natriumborhydrid in Gegenwart von Eisessig oder Trifluoressigsäure, vorzugsweise jedoch mit Phosphoroxychlorid/Natriumborhydrid, Diboran oder Diboran/Dimethylsulfid bei Temperaturen zwischen 0 und 50°C, vorzugsweise jedoch bei Temperaturen zwischen 10 und 25°C, durchgeführt. Bei der Reduktion kann eine gegebenenfalls im Rest $R_3$ vorhandene Carbonylfunktion gleichzeitig zu einer Methylengruppe mitreduziert werden.

d) Zur Herstellung von Verbindungen der Formel I, in der n die Zahl 0, $R_2$ ein Wasserstoffatom und $R_3$ eine Alkoxygruppe mit 1 bis 3 Kohlenstoffatomen, eine Alkoxygruppe mit 1 bis 6 Kohlenstoffatomen, die endständig durch eine Carboxy-, Alkoxycarbonyl-, Aminocarbonyl-, Alkylaminocarbonyl- -oder Dialkylaminocarbonylgruppe substituiert ist, oder eine Alkoxygruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, darstellen:

Umsetzung einer Verbindung der Formel

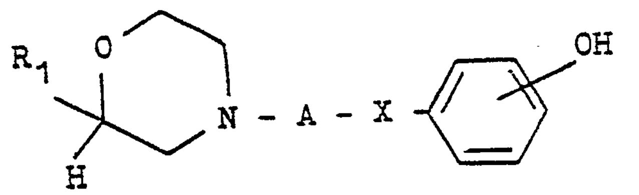

,(VIII)

in der
$R_1$, A und X wie eingangs definiert sind, mit einer Verbindung der Formel
$Z_3$ - $R_4$ ,(IX)
in der
$R_4$ eine Alkylgruppe mit 1 bis 3 Kohlenstoffatomen, eine Alkylgruppe mit 1 bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkylgruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, und
$Z_3$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, oder
$Z_3$ zusammen mit einem β-Wasserstoffatom des Restes $R_4$ ein Sauerstoffatom darstellen.

Die Umsetzung wird zweckmäßigerweise in einem Lösungsmittel wie Diethylether, Tetrahydrofuran, Dioxan, Methanol, Ethanol oder Dimethylformamid und vorzugsweise in Gegenwart eines säurebindenden Mittels wie Natriumhydroxid oder Kaliumtert.butylat, vorzugsweise jedoch in Gegenwart von Kaliumcarbonat oder Natriumhydrid, oder Pyridin, wobei eine organische Base wie Pyridin auch als Lösungsmittel dienen kann, oder zur Herstellung von 2-Hydroxy-ethoxy-Verbindungen der allgemeinen Formel I mit Ethylenoxid bei Temperaturen zwischen 0 und 100°C, vorzugsweise jedoch bei Temperaturen zwischen 20 und 80°C, durchgeführt.

e) Zur Herstellung von Verbindungen der Formel I, in der n die Zahl 0 darstellt und $R_3$ eine Alkoxycarbonyl-, Aminocarbonyl-, Alkylaminocarbonyl- oder Dialkylaminocarbonylgruppe darstellt oder enthält:
Umsetzung einer Verbindung der Formel

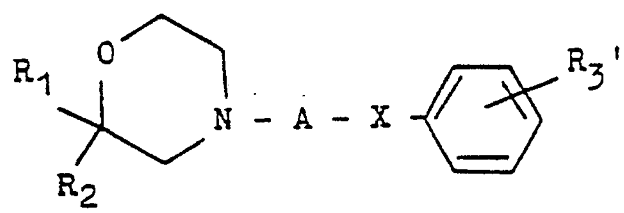

,(X)

in der
$R_1$, $R_2$, A und X wie eingangs definiert sind und
$R_3'$ eine Carboxygruppe oder eine Alkoxygruppe mit 1 bis 6 Kohlenstoffatomen, welche endständig durch eine Carboxygruppe substituiert ist, darstellt, oder deren reaktionsfähige Derivate wie beispielsweise deren Ester, mit einer Verbindung der Formel
H - $R_5$ ,(XI)
in der
$R_5$ eine Alkoxy-, Amino-, Alkylamino- oder Dialkylaminogruppe darstellt, wobei jeweils der Alkyl- oder Alkoxyteil 1 bis 3 Kohlenstoffatome enthalten kann.

Die Veresterung oder Amidierung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, besonders vorteilhaft jedoch in einem Überschuß einer eingesetzten Verbindung der allgemeinen Formel X, z.B. in Methanol, Ethanol, n-Propanol, Isopropanol, Ammoniak, Methylamin, Ethylamin, Dimethylamin oder Diethylamin, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/-N-Hydroxy-succinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 °C und 250 °C, vorzugsweise jedoch bei Temperaturen zwischen -10 °C und der Siedetemperatur des verwendeten Lösungsmittels, durchgeführt.

f) Zur Herstellung der Verbindungen der Formel I, in der n die Zahl 1 darstellt:
Umsetzung einer Verbindung der Formel

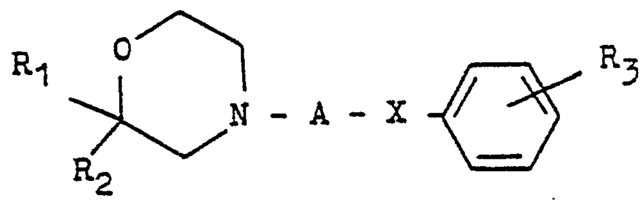

,(XII)

in der
$R_1$ bis $R_3$, A und X wie eingangs definiert sind, mit einer Peroxyverbindung und gegebenenfalls anschließende Hydrolyse.

Die Umsetzung wird zweckmäßigerweise in einem Lösungsmittel oder Lösungsmittelgemisch wie Methylenchlorid, Chloroform, Tetrahydrofuran, Acetonitril, Wasser, Methanol, Ethanol, Wasser/Ethanol, Eisessig oder Trifluoressigsäure mit einer Peroxyverbindung wie Wasserstoffperoxid, Perbenzoesäure oder m-Chlorperbenzoesäure bei Temperaturen zwischen 0 °C und der Siedetemperatur des verwendeten Lösungsmittels, vorzugsweise bei Temperaturen zwischen 60 und 80 °C, durchgeführt. Besonders vorteilhaft wird die Umsetzung durch Wasserstoffperoxid in Eisessig bei Temperaturen zwischen 60 und 80 °C, durchgeführt.

Die gegebenenfalls anschließende Hydrolyse wird zweckmäßigerweise in einem wässrigen Lösungsmittel, z.B. in Wasser, Isopropanol/Wasser, Tetrahydrofuran/Wasser oder Dioxan/Wasser, vorzugsweise in Gegenwart einer Säure wie Salzsäure oder Schwefelsäure bei Temperaturen zwischen 0 und 100 °C, vorzugsweise bei der Siedetemperatur des Reaktionsgemisches durchgeführt.

Bei den Umsetzungen a) bis g) können reaktionsfähige Gruppen während der Umsetzung durch übliche Schutzreste geschützt werden, diese werden nach der Umsetzung durch übliche Verfahren wieder abgespalten.

Als Schutzreste für eine Carboxygruppe kommen beispielsweise die Benzyl-, tert.Butyl-, Tetrahydropyranyl-, Trimethylsilyl-, Benzyloxymethyl-, 2-Chlorethyl- oder Methoxymethylgruppe oder eine Phenacylgruppe wie die Benzoylmethylgruppe,
als Schutzreste für eine Amino- oder Alkylaminogruppe die Acetyl-, Benzoyl-, tert.Butoxycarbonyl-, Benzyloxycarbonyl-, Ethoxycarbonyl- oder Benzylgruppe und
als Schutzreste für eine Hydroxygruppe beispielsweise die Trityl-, Fluorenylmethyloxycarbonyl-, Benzyloxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der Formel I, in der $R_3$ eine Alkoxycarbonyl-, Aminocarbonyl-, Alkylaminocarbonyl- oder Dialkylaminocarbonylgruppe darstellt oder enthält, so kann diese mittels Hydrolyse in eine entsprechende Verbindung der Formel I, in der $R_3$ eine Carboxygruppe darstellt oder enthält, übergeführt werden oder
eine Verbindung der Formel I, in der $R_3$ eine durch eine Carboxy-, Alkoxycarbonyl-, Aminocarbonyl-, Alkylaminocarbonyl- oder Dialkylaminocarbonylgruppe substituierte Alkoxygruppe darstellt, so kann diese mittels Reduktion durch ein geeignetes Metallhydrid in eine Verbindung der allgemeinen Formel I, in der der vorstehend erwähnte substituierte Alkoxyrest anstelle der Carbonylgruppe eine Methylengruppe enthält, übergeführt werden oder

eine Verbindung der Formel I, in der $R_3$ eine der eingangs erwähnten Alkoxygruppen darstellt, so kann diese mittels Etherspaltung in eine entsprechende Verbindung der Formel I, in der $R_3$ eine Hydroxygruppe oder eine durch eine Hydroxygruppe substituierte Alkoxygruppe darstellt, übergeführt werden.

Die nachträgliche Hydrolyse wird entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Äthanol, Äthanol/Wasser, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Die nachträgliche Reduktion wird in einem geeigneten Lösungsmittel wie Diethylether oder Tetrahydrofuran mit einem geeigneten Metallhydrid, z.B. mit Lithiumaluminiumhydrid, Diboran oder Diboran/Dimethylsulfid, vorzugsweise jedoch mit Natrium borhydrid in Gegenwart von Eisessig oder Trifluoressigsäure, bei Temperaturen zwischen 0 und 50 °C, vorzugsweise jedoch bei Temperaturen zwischen 10 und 25 °C, durchgeführt.

Die nachträgliche Etherspaltung wird in Gegenwart einer Säure wie Chlorwasserstoff, Bromwasserstoff, Schwefelsäure oder Bortribromid in einem geeigneten Lösungsmittel wie Methanol, Ethanol, Wasser/Isopropanol, Methylenchlorid, Chloroform oder Tetrachlorkohlenstoff bei Temperaturen zwischen -30 °C und der Siedetemperatur des Reaktionsgemisches durchgeführt.

Wie bereits eingangs erwähnt, können die neuen Verbindungen in Form ihrer Enantiomeren, Enantiomerengemische oder Racemate, oder sofern sie mindestens 2 asymmetrische Kohlenstoffatome enthalten, auch in Form ihrer Diastereomerenpaare bzw. Diastereomerenpaargemische vorliegen.

So lassen sich die erhaltenen Verbindungen der Formel I, welche nur ein optisch aktives Zentrum enthalten, nach an sich bekannten Methoden (siehe Allinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-o-Toluolweinsäure, Äpfelsäure, Mandelsäure, Camphersulfonsäure, Glutaminsäure, Asparaginsäure oder Chinasäure.

Desweiteren lassen sich die erhaltenen Verbindungen der Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der allgemeinen Formel I zwei optisch aktive Kohlenstoffatome, so erhält man die entsprechenden (R R′, S S′)- und (R S′, S R′)-Formen, oder 3 optisch aktive Zentren, so erhält man die entsprechenden (R R′ R″, S S′ S″), (R S′ R″, S R′ S″), (S R′ R″, R S′ S″)- und (R R′ S″, S S′ R″)-Formen.

Die als Ausgangsstoffe verwendeten Verbindungen, welche selbstverständlich auch in ihren optisch reinen Formen verwendet werden können, erhält man nach literaturbekannten Verfahren bzw. sind literaturbekannt. Diese liegen teilweise nur im Reaktionsgemisch vor und können daher teilweise nicht isoliert werden.

Beispielsweise erhält man eine als Ausgangsstoff verwendete Verbindung der Formel VIII oder X durch Alkylierung eines entsprechenden Morpholins und gegebenenfalls anschließende Abspaltung eines verwendeten Schutzrestes.

Die als Ausgangsstoffe verwendeten Verbindungen der Formel VII werden durch Umsetzung der in der EP-A-0.239.815 beschriebenen Verbindungen der Formel

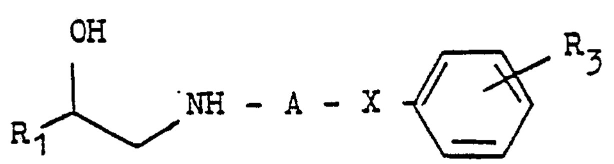

,(XIII)

in der

$R_1$, A, X und $R_3$ wie eingangs definiert sind, mit einem Halogenacetylchlorid oder einem entsprechenden Halogenessigester erhalten.

Die Umsetzung wird zweckmäßigerweise in einem Lösungsmittel wie Methylenchlorid, Tetrahydrofuran, Dioxan, Benzol oder Toluol oder in einem Überschuß des eingesetzten Acylierungsmittels gegebenenfalls in Gegenwart eines säurebindenden Mittels wie Kaliumcarbonat, einem Alkalimetallhydrid wie Natriumhydrid oder in Gegenwart einer tertiären organischen Base wie Triethylamin oder Pyridin, wobei die beiden letzteren gleichzeitig auch als Lösungsmittel dienen können, bei Temperaturen zwischen 0 und 100 °C, vorzugsweise jedoch bei Temperaturen zwischen der Raumtemperatur und der Siedetemperatur des Reaktionsgemisches durchgeführt.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Wie bereits eingangs erwähnt, weisen die neuen Verbindungen der Formel I, deren Enantiomeren, Enantiomerengemische oder Racemate, oder sofern sie mindestens 2 asymmetrische Kohlenstoffatome enthalten, auch deren Diastereomeren bzw. Diastereomerengemische und deren Säureadditionssalze, insbesondere für die pharmazeutische Anwendung deren physiologisch verträgliche Säureadditionssalze, wertvolle pharmakologische Eigenschaften auf, neben einer Hemmwirkung auf die Plättchenaggregation insbesondere eine Wirkung auf den Stoffwechsel, vorzugsweise eine blutzuckersenkende und körperfettreduzierende Wirkung, sowie eine senkende Wirkung auf die atherogenen $\beta$-Lipoproteine VLDL und LDL. Außerdem weisen einige der vorstehend erwähnten Verbindungen auch eine anabole Wirkung auf. - Hierbei hat sich beispielsweise bei den Verbindungen der Beispiele 3 bis 6 dasjenige Diastereomer als besonders bevorzugt erwiesen, bei dem das Protonenresonanzsignal der Methylgruppe, die dem Morpholinstickstoffatom benachbart ist, im $CDCl_3/CD_3OD$-NMR-Spektrum bei tieferem Feld liegt.

Die biologischen Eigenschaften der neuen Verbindungen wurden wie folgt untersucht:

1. Antidiabetische Wirkung:

Die antidiabetische Wirkung der erfindungsgemäßen Verbindungen läßt sich als eine blutzuckersenkende Wirkung bei Versuchstieren messen. Die zu untersuchenden Substanzen wurden dazu in 1,5%iger Methylzellulose suspendiert und weiblichen Mäusen eigener Zucht mittels Schlundsonde appliziert. 30 Minuten später wurde 1 g Glukose pro kg Körpergewicht in Wasser gelöst und subkutan appliziert. Weitere 30 Minuten später wurde aus dem retroorbitalen Venenplexus Blut entnommen. Aus dem Serum wurde Glukose mit der Hexokinase-Methode mit Hilfe eines Analysenphotometers bestimmt.

In der nachstehenden Tabelle sind die bei dieser Versuchsanordnung beobachteten Blutzuckersenkungen in % einer mitgeführten Kontrollgruppe zusammengestellt. Die statistische Auswertung erfolgt nach dem t-Test nach Student mit $p = 0,05$ als Signifikanzgrenze.

| Verbindung (Beispiel Nr.) | % Änderung vom Wert der Kontrollgruppe Dosis [mg/kg] 0,3 |
|---|---|
| 3 | - 50 |
| 4 | - 58 |
| 6 | - 65 |

2. Antiadipöse Wirkung:

Die antiadipöse Wirkung der erfindungsgemäßen Verbindungen wurde in zwei Versuchsanordnungen nachgewiesen:

a) In der ersten Versuchsanordnung wurde die Steigerung der Lipolyse am Anstieg des Glyzerins im Serum gemessen. Der Versuchsablauf ist identisch mit der zur Testung auf blutzuckersenkende Wirkung vorstehend beschriebenen Versuchsanordnung. Glyzerin wurde in einem kombinierten enzymatischkolorimetrischen Test mit Hilfe eines Analysenphotometers bestimmt. Die Ergebnisse sind in der nachfolgenden Tabelle in % einer mitgeführten Kontrollgruppe aufgeführt.

| Verbindung (Beispiel Nr.) | % Änderung vom Wert der Kontrollgruppe Dosis [mg/kg] 0,3 |
|---|---|
| 3 | + 262 |
| 4 | + 221 |
| 6 | + 314 |

b) In der zweiten Versuchsanordnung zur Erfassung antiadipöser Wirkung der erfindungsgemäßen Verbindungen wurde die Abnahme des Fettgewebes am Beispiel des Ovarfettgewebes gemessen. Die Verbindungen wurden zu diesem Zwecke Mäusen einmal täglich mittels Schlundsonde in 1,5%iger Methylzellulosesuspension appliziert. Am fünften Tag wurde das Ovarfettgewebe herauspräpariert und gewogen. In der nachfolgenden Tabelle sind die Ergebnisse in % einer mitgeführten Kontrollgruppe wiedergegeben.

| Verbindung (Beispiel Nr.) | % Änderung vom Wert der Kontrollgruppe Dosis: 1 [mg/kg] |
|---|---|
| 5 | - 41 |

3. Kardiale Nebenwirkungen:

Das Auftreten von unerwünschten Nebenwirkungen auf das Herz konnte für die erfindungsgemäßen Verbindungen für den therapeutisch angestrebten stoffwechselaktiven Dosisbereich ausgeschlossen werden. Als Nachweis diente die Messung der Herzfrequenz an Mäusen während der Testung auf blutzuckersenkende Wirkung (s.o.). Eine Stunde nach der oralen Applikation der Verbindungen wurde die Herzfrequenz mittels EKG-getriggertem Tachographen bestimmt. Die nachfolgende Tabelle gibt die Änderung der Herzfrequenz in % der Kontrollgruppe wieder.

| Verbindung (Beispiel Nr.) | % Änderung vom Wert der Kontrollgruppe | Dosis mg/kg |
|---|---|---|
| 3 | (+ 6) | 3,0 |
| 4 | ( 0 ) | 3,0 |
| 6 | (+ 4) | 3,0 |
| ( ) = nicht signifikant ($p > 0.05$) | | |

Desweiteren konnten bei den vorstehend beschriebenen Untersuchungen der erfindungsgemäßen Substanzen bei den applizierten Dosen keine toxischen Nebenwirkungen beobachtet werden. Diese sind daher gut verträglich.

Auf Grund ihrer pharmakologischen Eigenschaften eignen sich daher die neuen Verbindungen der Formel I, sowie deren physiologisch verträgliche Säureadditionssalze mit anorganischen oder organischen Säuren zur Behandlung sowohl des Diabetes mellitus als auch der Adipositas, insbesondere also zur Behandlung des adipösen Diabetikers. Außerdem können die neuen Verbindungen zur Prophylaxe und zur Behandlung atherosklerotischer Veränderungen der Gefäße, die vor allem bei Diabetikern und Obesen gehäuft auftreten, verwendet werden. Hierbei kann die erforderliche Dosis ganz auf den stoffwechselphysiologischen Bedarf des einzelnen Patienten abgestimmt werden, da diese über einen großen Dosisbereich frei von einer Herz/Kreislaufwirkung sind. Beim Erwachsenen liegen daher die Tagesdosis zwischen 1 und 3000 mg, vorzugsweise jedoch 1 bis 1000 mg, verteilt auf 1 bis 4 Dosen pro Tag. Hierzu lassen sich die obenerwähnten Verbindungen, gegebenenfalls in Kombination mit anderen Wirksubstanzen, in die üblichen galenischen Zubereitungsformen wie Pulver, Tabletten, Dragées, Kapseln, Suppositorien oder Suspensionen einarbeiten.

Desweiteren können die vorstehend erwähnten Verbindungen zur Behandlung fettsüchtiger Tiere wie

Hunde und als Folge ihrer körperfettreduzierenden (lipolytischen) Wirkung zur Reduktion unerwünschter Fetteinlagerungen bei der Mastzucht, also zur Verbesserung der Fleischqualität von Masttieren wie Schweinen, Rindern, Schafen und Geflügel eingesetzt werden. Bei den Tieren kann die Applikation der oben genannten Verbindungen oral oder auch nicht-oral, z.B. als Futterzusatz oder durch Injektion oder auch über implantierte Minipumpen erfolgen. Die Tagesdosis liegt hierbei zwischen 0,01 und 100 mg/kg, vorzugsweise jedoch zwischen 0,1 bis 10 mg/kg Körpergewicht.

Desweiteren können die vorstehend erwähnten Wirkstoffe als Leistungsförderer bei Tieren zur Förderung und Beschleunigung des Wachstums, der Milch- und Wollproduktion, sowie zur Verbesserung der Futterverwertung, der Schlachtkörperqualität und zur Verschiebung des Fleisch-Fett-Verhältnisses zugunsten von Fleisch eingesetzt werden. Die Wirkstoffe werden bei Nutz-, Zucht-, Zier- und Hobbytieren verwendet.

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthühner, Fische, wie z.B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier-und Aquarienfische, z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichtung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Tierart, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Tierart, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 10 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Futter beträgt normalerweise etwa 0,01 - 500 ppm, bevorzugt 0,1 - 50 ppm.

Die Wirkstoffe können als solche oder in Form von Prämixen oder Futterkonzentraten dem Futter zugesetzt werden.

So enthalten die erfindungsgemäßen Futtermittel neben dem Wirkstoff und gegebenenfalls neben einer üblichen Vitamin-Mineral-Mischung beispielsweise für Mastschweine Gerste, Weizennachmehl, Ackerbohnen, Raps-Extraktionsschrot und Futterfett, für Broiler Mais, Sojabohnenmehl, Fleischmehl, Futterfett und Sojaöl, für Rinder Zuckerrübenschnitzel Maiskleber, Malzkeime, Sojabohnenmehl, Weizen und Molasse sowie für Lämmer Gerste, Sojabohnenmehl, Mais und Melasse. Zu diesem Futter wird eine der eingangs erwähnten Verbindungen der Formel I als Wirkstoff in einer Konzentration von 0,01 bis 500 ppm, vorzugsweise jedoch von 0,1 bis 50 ppm, zugemischt, wobei die Zumischung vorzugsweise in Form einer Wirkstoffvormischung erfolgt. Diese Vormischung enthält beispielsweise pro 10 g 10 mg Wirkstoff zweckmäßigerweise in 9,99 g Maisstärke.

Die nachfolgenden Beispiele sollen die Erfindung näher erläutern:

Beispiel A

5-Brom-3-acetyl-pyridin

44,2 g (0,276 Mol) Malonsäurediethylester, 19,9 g (0,432 Mol) absolutes Ethanol und 7 g (0,288 Mol) Magnesiumspäne werden in 254 ml Ether und 1,27 ml Tetrachlorkohlenstoff für 18 Stunden unter Rühren und unter Rückfluß erhitzt. Zu dieser Lösung werden 55,3 g (0,25 Mol) 5-Bromnicotinsäurechlorid in 170 ml absolutem Tetrahydrofuran langsam zugetropft und für eine Stunde weiter unter Rückfluß erhitzt. Die Reaktionsmischung wird im Eisbad abgekühlt und vorsichtig mit 508 ml 2N Schwefelsäure versetzt. Die organische Phase wird abgetrennt, über Natriumsulfat getrocknet und eingedampft. Der Rückstand wird in 83 ml Wasser, 127 ml Eisessig und 16,6 ml konzentrierter Schwefelsäure aufgenommen und über 18 Stunden unter Rückfluß gekocht. Die Reaktionslösung wird eingedampft, der Rückstand mit 200 ml Wasser verdünnt und mit Natronlauge auf pH 8 gestellt. Anschließend wird mit Chlorofrom extrahiert, über Natriumsulfat getrocknet und eingedampft.
Ausbeute: 15,2 g (30,4 % der Theorie),
Schmelzpunkt: 78-80 °C

| | | | | | | | | |
|---|---|---|---|---|---|---|---|---|
| Ber.: | C | 42,03 | H | 3,01 | N | 7,00 | Br | 39,95- |
| Gef.: | | 42,20 | | 3,30 | | 6,82 | | 40,06 |

Beispiel B

5-Brom-3-bromacetyl-pyridin-hydrobromid

4 g (0,02 Mol) 5-Brom-3-acetyl-pyridin werden in 50 ml Chloroform gelöst, 5 Tropfen Eisessig/Bromwasserstoff (35%ig) zugesetzt und dann zum Sieden erhitzt. Zu dieser kochenden Lösung werden unter Rühren 3,2 g (0,02 Mol) Brom in 30 ml Chlo roform über 5 Stunden lang zugetropft. Nach Abkühlen auf Raumtemperatur wird das gelbe Festprodukt abgesaugt.
Ausbeute: 5 g (70 % der Theorie),
Schmelzpunkt: 225 °C (Zers.)

| | | | | | | | | |
|---|---|---|---|---|---|---|---|---|
| Ber.: | C | 23,35 | H | 1,67 | N | 3,88 | Br | 66,60 |
| Gef.: | | 23,30 | | 1,73 | | 3,81 | | 66,40 |

Beispiel C

2-(6-Chlor-pyridin-2-yl)-morpholin-5-on

6 g (0,0347 Mol) 2-Hydroxy-2-(2-chlor-pyridin-6-yl)-ethanamin werden in 160 ml absolutem Toluol gelöst und mit 2,2 g (0,047 Mol) 50%igem Natriumhydrid in Öl versetzt und 40 Minuten bei Raumtemperatur gerührt. Anschließend werden 4,16 g (0,034 Mol) Chloressigsäureethylester zugetropft. Das Reaktionsgemisch wird über 18 Stunden bei Raumtemperatur gerührt und dann vorsichtig mit 10 ml Ethanol und 30 ml Wasser versetzt und anschließend mit verdünnter Salzsäure angesäuert. Die Reaktionslösung wird mit Ammoniak alkalisch gestellt und mit Methylenchlorid extrahiert. Die organische Phase wird über Natriumsulfat getrocknet, eingeengt und der Rückstand über eine Kieselgelsäule mit Essigester als Eluens gereinigt.
Ausbeute: 1 g (14 % der Theorie),

| | | | | | | |
|---|---|---|---|---|---|---|
| Ber.: | C | 50,83 | H | 4,26 | N | 13,17 |
| Gef.: | | 50,55 | | 4,11 | | 13,30 |

Beispiel D

2-(6-Chlor-pyridin-2-yl)-morpholin

1 g (0,0047 Mol) 2-(6-Chlor-pyridin-2-yl)-morpholin-5-on werden in 20 ml absolutem Tetrahydrofuran gelöst, unter Rüh ren bei Raumtemperatur 5 ml einer 1 molaren Lösung von Boran in Tetrahydrofuran zugesetzt und die Reaktionslösung für 60 Minuten gerührt. Anschließend wird portionsweise noch soviel Boran/Tetrahydrofuran-Lösung zugesetzt bis laut Dünnschichtchromatogramm alles Ausgangsmaterial umgesetzt war. Anschließend wird das Lösungsmittel abgezogen, der Rückstand in 20 ml Methanol über 18 Stunden gerührt und dann eingedampft. Dieser Rückstand wird in 20 ml 2n Salusäure aufgenommen und für 2 Stunden bei 50° C gerührt. Die saure Lösung wird im Eisbad abgekühlt, mit Ammoniaklösung alkalisch gestellt und mit Methylenchlorid extrahiert. Die organische Phase wird über Natriumsulfat getrocknet, eingeengt und der Rückstand über eine Kieselgelsäule mit Methanol als Eluens gereinigt.
Ausbeute: 0,4 g (43 % der Theorie),

| | | | | | | | | |
|---|---|---|---|---|---|---|---|---|
| Ber.: | C | 54,42 | H | 5,57 | N | 14,10 | Cl | 17,84 |
| Gef.: | | 54,30 | | 5,70 | | 13,90 | | 18,01 |

Beispiel E

N-[2-(4-(2-Hydroxyethoxy)-phenyl)-1-methylethyl]-2-hydroxy-2-(6-chlor-pyridin-2-yl)-ethanamin

21.2 g (0,122 Mol) 2-Hydroxy-2-(6-chlor-pyridin-2-yl)-ethanamin und 21 g (0,108 Mol) 1-(4-(2-Hydroxyethoxy)-phenyl)-propan-2-on werden in 500 ml absolutem Methanol gelöst, mit 7,3 g (0,122 Mol) Essigsäure und 7,2 g (0,114 Mol) Natriumcyanborhydrid versetzt und über Nacht bei Raumtemperatur gerührt. Anschließend werden 300 ml Methanol im Vakuum abdestilliert, mit 400 ml Wasser verdünnt, mit Salzsäure angesäuert und dann dreimal mit je 250 ml Methylenchlorid extrahiert. Die wäßrige Phase wird dann mit Ammoniak alkalisch gestellt, mit Methylenchlorid extrahiert. Die dabei erhaltene organische Phase wird über Natriumsulfat getrocknet und eingeengt, wobei das anfänglich erhaltene Öl nach kurzer Zeit kristallisiert.
Ausbeute: 23 g (60,7 % der Theorie),
Schmelzpunkt: 100-102° C

| | | | | | | | | |
|---|---|---|---|---|---|---|---|---|
| Ber.: | C | 61,62 | H | 6,60 | N | 7,98 | Cl | 10,10 |
| Gef.: | | 61,48 | | 6,64 | | 7,86 | | 10,28 |

Beispiel F

N-[2-(4-(2-Hydroxyethoxy)-phenyl)-1-methylethyl]-2-(6-chlor-pyridin-2-yl)-morpholin-5-on

1 g (0,0285 Mol) N-[2-(4-(2-Hydroxyethoxy)-phenyl)-1-methylethyl]-2-hydroxy-2-(6-chlor-pyridin-2-yl)-ethanamin wird in 40 ml Methylenchlorid gelöst, 0,28 g (0,00285 Mol) Triäthylamin zugesetzt und dann unter Rühren im Eisbad 0,322 g (0,00285 Mol) Chloracetylchlorid hinzugetropft. Die Reaktionslösung wird für 40 Minuten bei Raumtemperatur gerührt und eingedampft. Der Rückstand wird in 10 ml Dimethylformamid gelöst, mit 110 mg (0,00234 Mol) 50-55%igem Natriumhydrid in Öl versetzt und 30 Minuten bei Raumtemperatur gerührt. Dann vorsichtig mit 30 ml Wasser zersetzt, angesäuert mit 2N Salzsäure und anschließend alkalisch mit 2N Ammoniak gestellt. Das Produkt wird extahiert mit Methylenchlorid, getrocknet über Natriumsulfat und eingedampft. Der Rückstand wird gereinigt über eine Kieselgelsäule mit Essigester als Eluens.
Ausbeute: 0,7 g (76,5 % der Theorie),

| | | | | | | | | |
|---|---|---|---|---|---|---|---|---|
| Ber.: | C | 61,45 | H | 5,93 | N | 7,16 | Cl | 9,06 |
| Gef.: | | 61,30 | | 5,88 | | 7,30 | | 8,88 |

Beispiel 1

N-[2-(4-Carbomethoxymethoxyphenyl)-1-methylethyl]-2-hydroxy-2-(5-brom-pyridin-3-yl)-morpholin

3,8 g (0,0142 Mol) N-(2-Hydroxyethyl)-2-(4-carbomethoxyphenyl)-1-methyl-ethylamin werden mit 5 g (0,0142 Mol) 5-Brom-3-bromacetyl-pyridin-hydrobromid und 5,6 g (0,056 Mol) Kaliumhydrogencarbonat in 300 ml Aceton 2 Stunden lang bei Raumtemperatur gerührt. Anschließend werden 1,4 g (0,014 Mol) Triäthylamin zugesetzt und bei 30-40 °C weitere 60 Minuten gerührt. Nach Absaugen und Einengen des Filtrats wird der ölige Rückstand über eine Kieselgelsäule mit Toluol/Essigester (60/40) als Eluens gereinigt
Ausbeute: 1,4 g Öl (21 % der Theorie),

| | | | | | | |
|---|---|---|---|---|---|---|
| Ber.: | C | 54,20 | H | 5,42 | N | 6,02 |
| Gef.: | | 54,07 | | 5,31 | | 6,19 |

Laut $^1$H-NMR-Spektrum (400 MHz, $CDCl_3/CD_3OD$) liegt ein ca. 1:1-Gemisch der Diastereomerenpaare vor.
$\delta$ = 1,07 ppm (d, 3H)
$\delta$ = 0,98 ppm (d, 3H)

Beispiel 2

N-[2-(4-Carboethoxymethoxyphenyl)-1-methylethyl]-2-hydroxy-2-(5-brom-pyridin-3-yl)-morpholin

Hergestellt analog Beispiel 1 durch Umsetzung von 1,0 g (0,007 Mol) N-(2-Hydroxyethyl)-2-(4-carboethoxyphenyl)-1-methyl-ethylamin mit 2,5 g (0,007 Mol) 5-Brom-3-bromacetyl-pyridin-hydrobromid, 2,8 g (0,028 Mol) Kaliumhydrogencarbonat und 0,7 g (0,007 Mol) Triethylamin in 150 ml Aceton.
Ausbeute: 1 g Öl (30 % der Theorie),

| | | | | | | |
|---|---|---|---|---|---|---|
| Ber.: | C | 55,12 | H | 5,68 | N | 5,85 |
| Gef.: | | 54,98 | | 5,57 | | 5,83 |

Laut $^1$H-NMR-Spektrum (400 MHz, $CDCl_3/CD_3OD$) liegt ein ca. 1:1-Gemisch der Diastereomerenpaare vor.
$\delta$ = 1,07 ppm (d, 3H)
$\delta$ = 0,98 ppm (d, 3H)

Beispiel 3

N-[2-(4-Carbomethoxymethoxyphenyl)-1-methylethyl]-2-(6-chlor-pyridin-2-yl)-morpholin

0,35 g (0,00176 Mol) 2-(6-Chlor-pyridin-2-yl)-morpholin und 0,39 g (0,00176 Mol) 1-(4-Carbomethoxymethoxyphenyl)-propan-2-on werden in 20 ml Methanol gelöst, mit 0,1 g (0,00176 Mol) Essigsäure und 0,11 g (0,00176 Mol) Natriumcyanborhydrid versetzt und 20 Minuten bei Raumtemperatur gerührt. Anschließend wird mit Eiswasser versetzt, mit Salzsäure angesäuert, mit Ammoniaklösung alkalisch gestellt, das Produkt mit Methylenchlorid extrahiert, mit Natriumsulfat getrocknet und im Vakuum eingeengt. Der Rückstand wird über eine Kieselgelsäule mit Toluol/Essigester (50:50) als Eluens gereinigt.
Ausbeute: 0,6 g Öl (84 % der Theorie),

| | | | | | | |
|---|---|---|---|---|---|---|
| Ber.: | C | 62,29 | H | 6,22 | N | 6,92 |
| Gef.: | | 62,35 | | 6,17 | | 6,74 |

Laut $^1$H-NMR-Spektrum (400 MHz, $CDCl_3/CD_3OD$) liegt ein ca. 1:1-Gemisch der Diastereomerenpaare vor.
$\delta$ = 0,988 ppm (d, 3H)
$\delta$ = 0,958 ppm (d, 3H)

## Beispiel 4

### N-[2-(4-Carboxymethoxyphenyl)-1-methylethyl]-2-(6-chlor-pyridin-2-yl)-morpholin

0,2 g (0,000453 Mol) N-[2-(4-Carbomethoxymethoxyphenyl)-1-methylethyl]-2-(6-chlor-pyridin-2-yl)-morpholin werden in 1 ml Methanol gelöst, mit 1 ml Dioxan und 2 ml In Natronlauge versetzt und für 30 Minuten bei Raumtemperatur gerührt. Dann wird mit 1,8 ml In Salzsäure neutralisiert, mit Chloroform extrahiert, über Natriumsulfat getrocknet und eingeengt. Der Rückstand wird mit Ether verrieben und abgesaugt.
Ausbeute: 0,12 g (68 % der Theorie),
Schmelzpunkt: ab 60° C (Zers.)

| | | | | | | |
|---|---|---|---|---|---|---|
| Ber.: | C | 61,45 | H | 5,93 | N | 7,17 |
| Gef.: | | 61,28 | | 5,79 | | 7,08 |

Laut $^1$H-NMR-Spektrum (400 MHz, $CDCl_3/CD_3OD$) liegt ein ca. 1:1-Gemisch der Diastereomerenpaare vor.
$\delta$ = 1,11 ppm (d, 3H)
$\delta$ = 1,12 ppm (d, 3H)

## Beispiel 5

### N-[2-(4-(2-Hydroxyethoxy)-phenyl)-1-methylethyl]-2-(6-chlor-pyridin-2-yl)-morpholin

0,7 g N-[2-(4-(2-Hydroxyethoxy)-phenyl)-1-methylethyl]-2-(6-chlor-pyridin-2-yl)-morpholin-5-on werden in 20 ml Tetrahydrofuran gelöst und über 3 Stunden bei Raumtemperatur 3 mal 4 ml einer 1 molaren Boran/Tetrahydrofuran-Lösung zugegeben. Anschließend wird mit 30 ml Aceton versetzt und eingedampft. Der Rückstand wird in 100 ml Methanol gelöst und 20 Stunden gerührt. Das Lösungsmittel wird im Vakuum abdestilliert und der Rückstand über eine Kieselgeläsule mit Essigester als Eluens gereinigt.
Ausbeute: 0,6 g Öl (89 % der Theorie),

| | | | | | | |
|---|---|---|---|---|---|---|
| Ber.: | C | 63,73 | H | 6,69 | N | 7,43 |
| Gef.: | | 63,84 | | 6,58 | | 7,32 |

Laut $^1$H-NMR-Spektrum (400 MHz, $CDCl_3$) liegt ein ca. 1:1-Gemisch der Diastereomerenpaare vor.
$\delta$ = 0,962 ppm (d, 3H)
$\delta$ = 0,930 ppm (d, 3H)

Beispiel 6

N-[2-(4-(2-Hydroxyethoxy)-phenyl)-1-methylethyl]-2-(6-chlor-pyridin-2-yl)-morpholin

0,1 g (0,000246 Mol) N-[2-(4-Carbomethoxymethoxyphenyl)-1-methylethyl]-2-(6-chlor-pyridin-2-yl)-morpholin werden in 10 ml absolutem Tetrahydrofuran gelöst und im Laufe von einer Stunde zweimal je 5,5 mg (0,0005 Mol) Lithiumborhydrid zugegeben. Die Reaktionslösung wird 20 Stunden bei Raumtemperatur gerührt, eingedampft und der Rückstand vorsichtig mit 15 ml Methanol und 0,5 ml konzentrierter Salzsäure versetzt. Anschließend wird mit Ammoniaklösung alkalisch gestellt mit Methylenchlorid extrahiert, über Natriumsulfat getrocknet und im Vakuum eingeengt. Der Rückstand wird über eine Kieselgelsäule mit Toluol/Essigester (50:50) als Eluens gereinigt.
Ausbeute: 0,075 g Öl (81 % der Theorie),

| | | | | | | |
|---|---|---|---|---|---|---|
| Ber.: | C | 63,73 | H | 6,69 | N | 7,43 |
| Gef.: | | 63,69 | | 6,55 | | 7,40 |

Laut $^1$H-NMR-Spektrum (400 MHz, $CDCl_3/CD_3OD$) liegt ein ca. 1,3:1-Gemisch der Diastereomerenpaare vor.
$\delta$ = 0,990 ppm (d, 3H)
$\delta$ = 0,965 ppm (d, 3H)

Beispiel 7

N-[2-(4-Carbomethoxymethoxyphenyl)-1-methylethyl]-2-(pyridin-3-yl)-morpholin

1,80 g (6,73 mmol) N-(2-Hydroxyethyl)-2-(4-carbomethoxymethoxyphenyl)-1-methyl-ethylamin werden mit 1,74 g (6,2 mmol) 3-Bromacetyl-pyridin-hydrobromid und 2,44 g (24,5 mmol) Kaliumhydrogencarbonat in 50 ml Aceton 24 Stunden bei Raumtemperatur gerührt. Die anorganischen Salze werden abfiltriert und das Filtrat am Rotationsverdampfer eingedampft. Der erhaltene Rückstand wird in 20 ml Trifluoressigsäure gelöst und mit 1,39 ml (8,68 x $10^{-3}$ Mol) Triethylsilan unter Eiskühlung reduziert. Nach einer Stunde unter Eiskühlung und 12 Stunden bei Raumtemperatur wird die erhaltene Lösung eingedampft. Der Rückstand mit Eis versetzt und mit konzentriertem Ammoniak alkalisch gestellt. Die wässrige Lösung wird 3 x mit Methylenchlorid extrahiert, die organischen Phasen vereinigt, getrocknet, filtriert und am Rotationsverdampfer eingedampft. Das erhaltene Öl wird über eine Kieselgelsäule mit Essigester als Eluens gereinigt. Das Hydrochlorid wird aus Isopropanol und Ether mit etherischer Salzsäure gefällt.
Ausbeute: 0,5 g (18 % der Theorie),
Schmelzpunkt: 190-192 °C (Zers.)

| | | | | | | |
|---|---|---|---|---|---|---|
| Ber.: | C | 56,89 | H | 6,37 | N | 6,32 |
| Gef.: | | 56,79 | | 6,22 | | 6,23 |

Laut $^1$H-NMR-Spektrum (400 MHz, $CDCl_3/CD_3OD$) liegt ein ca. 1:1-Gemisch der Diastereomerenpaare vor.

Beispiel 8

N-[2-(4-Carbomethoxymethoxyphenyl)-1-methylethyl]-2-hydroxy-2-(pyrazin-2-yl)-morpholin

Hergestellt analog Beispiel 1 durch Umsetzung von N-(2-Hydroxyethyl)-2-(4-carbomethoxymethoxyphenyl)-1-methyl-ethylamin mit 2-Bromacetyl-pyrazin-hydrobromid in Gegenwart von Kaliumhydrogencarbonat. Die Reinigung erfolgt über Kieselgel mit Essigester/Cyclohexan (9:1) als Eluens.
Ausbeute: 46 % der Theorie, Öl

| | | | | | | |
|---|---|---|---|---|---|---|
| Ber.: | C | 62,00 | H | 6,50 | N | 10,85 |
| Gef.: | | 61,90 | | 6,61 | | 10,76 |

Laut $^1$H-NMR-Spektrum (400 MHz, $CDCl_3/CD_3OD$) liegt ein ca. 1:1-Gemisch der Diastereomerenpaare vor.
$\delta$ = 1,01 ppm (d, 3H)
$\delta$ = 1,03 ppm (d, 3H)

Beispiel 9

N-[2-(4-Carbomethoxymethoxyphenyl)-1-methylethyl]-2-hydroxy2-(pyridin-2-yl)-morpholin

Hergestellt analog Beispiel 1 durch Umsetzung von N-(2-Hydroxymethyl)-2-(4-carbomethoxymethoxyphenyl)-1-methyl-ethylamin mit 2-Bromacetyl-pyridin-hydrobromid in Gegenwart von Kaliumhydrogencarbonat. Die Reinigung erfolgt über Kieselgel mit Toluol/Essigester (1:1) als Eluens.
Ausbeute: 11 % der Theorie, Öl

| | | | | | | |
|---|---|---|---|---|---|---|
| Ber.: | C | 65,27 | H | 6,78 | N | 7,25 |
| Gef.: | | 65,34 | | 6,89 | | 7,13 |

Laut $^1$H-NMR-Spektrum (400 MHz, $CDCl_3/CD_3OD$) liegt ein ca. 1:1-Gemisch der Diastereomerenpaare vor.
$\delta$ = 1,00 ppm (d, 3H)
$\delta$ = 1,02 ppm (d, 3H)

Beispiel 10

N-[2-(4-Carbomethoxymethoxyphenyl)-1-methylethyl]-2-hydroxy-2-(pyridin-4-yl)-morpholin

Hergestellt analog Beispiel 1 durch Umsetzung von N-(2-Hydroxyethyl)-2-(4-carbomethoxymethoxyphenyl)-1-methyl-ethylamin mit 4-Bromacetyl-pyridin-hydrobromid in Gegenwart von Kaliumhydrogencarbonat. Die Reinigung erfolgt über Kieselgel mit Essigester als Eluens.
Ausbeute: 43 % der Theorie, Öl

| | | | | | | |
|---|---|---|---|---|---|---|
| Ber.: | C | 65,27 | H | 6,78 | N | 7,25 |
| Gef.: | | 65,30 | | 6,73 | | 7,22 |

Laut $^1$H-NMR-Spektrum (400 MHz, $CDCl_3/CD_3OD$) liegt ein ca. 1:1-Gemisch der Diastereomerenpaare vor.
$\delta$ = 0,979 ppm (d, 3H)
$\delta$ = 1,000 ppm (d, 3H)

Beispiel 11

N-[2-(4-(2-Hydroxyethoxy)-phenyl)-1-methylethyl]-2-(6-chlor-pyridin-2-yl)-morpholin-N-oxid

0,13 g (0,34 mmol) eines 1:1-Gemisches von N-[2-(4-(2-Hydroxyethoxy)-phenyl)-1-methylethyl]-2-(6-chlor-pyridin-2-yl)-morpholin werden in 2 ml Essigsäure gelöst und mit 40 mg Wasserstoffsuperoxid für 1,5 Stunden auf 85 °C erhitzt und anschließend zur Trockene eingeengt. Der Rückstand wird mit Eis versetzt, mit Ammoniak alkalisch gestellt und mit Methylenchlorid extrahiert. Die organische Phase wird getrocknet und bei 20 °C zur Trockene eingeengt. Der Rückstand wird über eine Kieselgelsäule mit Methylenchlorid/Methanol (8:2) als Eluens gereinigt.

Ausbeute: 60 mg (45 % der Theorie),
Schmelzpunkt: 94 °C (Zers.)

| | | | | | | |
|---|---|---|---|---|---|---|
| Ber.: | C | 61,14 | H | 6,41 | N | 7,13 |
| Gef.: | | 61,22 | | 6,50 | | 7,08 |

Laut $^1$H-NMR-Spektrum (400 MHz, DMSO/$CD_3OD$) liegt die Verbindung als reines Diastereomer A vor.
$\delta$ = 1,18 ppm (d, 3H)

Beispiel 12

N-[2-(4-(2-Hydroxyethoxy)-phenyl)-1-methylethyl]-2-(6-chlor-pyridin-2-yl)-morpholin

Diastereomerenpaar A:

3 g eines 1:1-Gemisches der Diastereomerenpaare von N-[2-(4-(2-Hydroxyethoxy)-phenyl)-1-methylethyl]-2-(6-chlor-pyridin-2-yl)-morpholin werden in Ether gelöst und das Hydrochlorid wird mit Diisopropylether/Salzsäure gefällt. Das Salz wird in 50 ml Aceton gelöst und zur Trockene eingedampft. Der Rückstand wird mit 30 ml Aceton aufgekocht und auf Raumtemperatur abgekühlt, wobei nach Reiben Kristallisation erfolgt.
Ausbeute: 1,3 g (43 % der Theorie),
Schmelzpunkt des Hydrochlorids: 196-198 °C

| | | | | | | | | |
|---|---|---|---|---|---|---|---|---|
| Ber.: | C | 58,11 | H | 6,34 | N | 6,78 | Cl | 17,16 |
| Gef.: | | 57,93 | | 6,47 | | 6,78 | | 17,03 |

$^1$H-NMR-Spektrum der Base (400 MHz, $CDCl_3$):
$\delta$ = 4,610 ppm (dd, >CH-OH)

Diastereomerenpaar B:

Die Mutterlauge wird zur Trockene eingedampft und der erhaltene Rückstand im Vakuum getrocknet.
Ausbeute: 1,7 g (56 % der Theorie),
Schmelzpunkt des Hydrochlorids: ab 70 °C (Zers.)

| | | | | | | | | |
|---|---|---|---|---|---|---|---|---|
| Ber.: | C | 58,11 | H | 6,34 | N | 6,78 | Cl | 17,16 |
| Gef.: | | 58,00 | | 6,21 | | 6,70 | | 17,20 |

$^1$H-NMR-Spektrum der Base (400 MHz, $CDCl_3$):
$\delta$ = 4,640 ppm (dd, >CH-OH)

Beispiel I

Dragée mit 10 mg N-[2-(4-(2-Hydroxyethoxy)-phenyl)-1-methyl-ethyl]-2-(6-chlor-pyridin-2-yl)-morpholin

| Zusammensetzung: | | |
|---|---|---|
| 1 Dragée enthält: | | |
| (1) | Wirksubstanz | 10,0 mg |
| (2) | Milchzucker | 69,0 mg |
| (3) | Maisstärke | 35,0 mg |
| (4) | Polyvinylpyrrolidon | 5,0 mg |
| (5) | Magnesiumstearat | 1,0 mg |
| | | 120,0 mg |

Herstellung:

(1) + (2) + (3) werden gemischt und mit (4) in einer wäßrigen Lösung befeuchtet. Die feuchte Masse wird durch ein Sieb mit 1,6 mm Maschenweite geschlagen und bei 45 °C im Umlufttrockenschrank getrocknet. Das trockene Granulat wird durch ein Sieb mit 1 mm Maschenweite gegeben und mit (5) gemischt. Die fertige Mischung wird zu Dragéekernen verpreßt.

| | |
|---|---|
| Kerngewicht: | 120,0 mg |
| Durchmesser: | 7,0 mm |
| Wölbungsradius: | 6,0 mm |

Die so hergestellten Dragéekerne werden auf bekannte Weise mit einer Schicht überzogen, die im Wesentlichen aus Zucker und Talkum besteht. Diese Schicht kann auch Farbauszüge enthalten. Die fertigen Dragées werden mit Wachs poliert.
Dragéegewicht: 180,0 mg

Beispiel II

Dragée mit 50 mg N-[2-(4-(2-Hydroxyethoxy)-phenyl)-1-methyl-ethyl]-2-(6-chlor-pyridin-2-yl)-morpholin

| Zusammensetzung: | | |
|---|---|---|
| 1 Dragée enthält: | | |
| (1) | Wirksubstanz | 50,0 mg |
| (2) | Milchzucker | 110,8 mg |
| (3) | Maisstärke | 50,0 mg |
| (4) | Polyvinylpyrrolidon | 8,0 mg |
| (5) | Magnesiumstearat | 1,2 mg |
| | | 220,0 mg |

Herstellung:

Die Herstellung erfolgt analog Beispiel I.

| | |
|---|---|
| Kerngewicht: | 220,0 mg |
| Durchmesser: | 9,0 mm |
| Wölbungsradius: | 8,0 mm |
| Dragéegewicht: | 300,0 mg |

Beispiel III

Tabletten mit 150 mg N-[2-(4-(2-Hydroxyethoxy)-phenyl)-1-methylethyl]-2-(6-chlor-pyridin-2-yl)-morpholin

| Zusammensetzung: | | |
|---|---|---|
| 1 Tablette enthält: | | |
| (1) | Wirksubstanz | 150,0 mg |
| (2) | Milchzucker | 86,0 mg |
| (3) | Maisstärke | 50,8 mg |
| (4) | Mikrokristalline Zellulose | 25,0 mg |
| (5) | Polyvinylpyrrolidon | 7,0 mg |
| | Magnesiumstearat | 1,2 mg |
| | | 320,0 mg |

Herstellung:

(1) + (2) + (3) + (4) + (5) werden gemischt und mit Wasser befeuchtet. Die feuchte Masse wird durch ein Sieb mit 1,6 mm Maschenweite geschlagen und bei 45° C getrocknet. Das trockene Granulat wird nochmals durch dasselbe Sieb gegeben und mit (6) gemischt. Aus der fertigen Mischung werden Tabletten gepreßt.

| | |
|---|---|
| Tablettengewicht: | 320,0 mg |
| Durchmesser: | 10,0 mm |

Die Tabletten werden mit einer Teilkerbe versehen, um die Halbierung zu ermöglichen.

Beispiel IV

Hartgelatinekapseln zu 100 mg N-[2-(4-(2-Hydroxyethoxy)-phenyl)-1-methylethyl]-2-(6-chlor-pyridin-2-yl)-morpholin

| Zusammensetzung: | | |
|---|---|---|
| 1 Kapsel enthält: | | |
| Kapselhülle: Hartgelatinekapseln Größe 3 | | |
| Kapselinhaltsstoffe: | | |
| (1) | Wirksubstanz | 100,0 mg |
| (2) | Lactose x 1$H_2O$ | 38,0 mg |
| (3) | Maisstärke getrocknet | 60,0 mg |
| (4) | Magnesiumstearat | 2,0 mg |
| | Kapselfüllgewicht: | 200,0 mg |
| (5) | Dest. Wasser | q.s. |

Herstellung:

Ein kleiner Teil Lactose wird ca. 10 %ig in dest. Wasser gelöst (Granulierflüssigkeit). Der Wirkstoff, die

restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

Beispiel V

Hartgelatinekapseln zu 200 mg N-[2-(4-(2-Hydroxyethoxy)-phenyl)-1-methylethyl]-2-(6-chlor-pyridin-2-yl)-morpholin

| | | |
|---|---|---|
| Zusammensetzung: | | |
| Kapselhülle: Hartgelatinekapseln Größe 1 | | |
| Kapselinhaltsstoffe: | | |
| (1) | Wirksubstanz | 200,0 mg |
| (2) | Lactose x 1 $H_2O$ | 47,0 mg |
| (3) | Maisstärke getrocknet | 70,0 mg |
| (4) | Magnesiumstearat | 3,0 mg |
| | Kapselfüllgewicht: | 320,0 mg |
| (5) | Dest. Wasser | q.s. |

Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

Beispiel VI

Alleinfutter II für Mastschweine

| | |
|---|---|
| Gerste | 370 g/kg |
| Weizennachmehl | 200 g/kg |
| Maniokmehl | 135 g/kg |
| Ackerbohnen | 100 g/kg |
| Raps-Extraktionsschrot | 100 g/kg |
| Futterfett lysinreiches Mineralfutter für Schweine | 65 g/kg |
| lysinreiches Mineralfutter für Schweine | 20 g/kg |
| Wirkstoff-Vormischung | 10 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel VII

Mastfutter II für Broiler

| | |
|---|---|
| Mais | 625 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-/Mineralstoffmischung | 5 g/kg |
| Wirkstoff-Vormischung | 10 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel VIII

Kraftfutter für Rinder

| | |
|---|---|
| Zuckerrübenschnitzel | 600,0 g/kg |
| Maiskleber | 100,0 g/kg |
| Malzkeime | 50,0 g/kg |
| Sojabohnenmehl | 35,0 g/kg |
| Weizen | 110,0 g/kg |
| Melasse | 60,0 g/kg |
| Futterphosphate | 12,0 g/kg |
| Calciumcarbonat | 2,5 g/kg |
| Salz | 5,0 g/kg |
| Mineralstoffe | 10,0 g/kg |
| Vitamin-Vormischung | 5,5 g/kg |
| Wirkstoff-Vormischung | 10,0 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel IX

Mastfutter für Lämmer

| | |
|---|---|
| Gerste | 690 g/kg |
| Sojabohnenmehl | 100 g/kg |
| Mais | 150 g/kg |
| Melasse | 30 g/kg |
| Vitamin-/Mineralstoffmischung | 20 g/kg |
| Wirkstoffvormischung | 10 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

**Ansprüche**

1. Morpholine und Morpholin-N-oxide der Formel

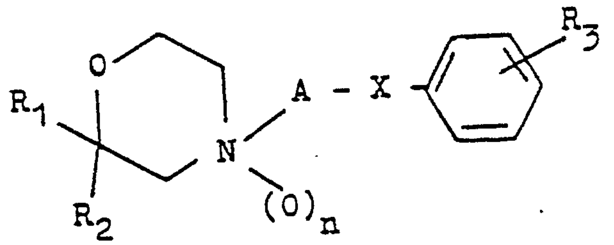

,(I)

in der
A eine gegebenenfalls durch Methyl- oder Ethylgruppen mono-oder disubstituierte n-Alkylengruppe mit 2 oder 3 Kohlenstoffatomen,
X eine Bindung oder ein Sauerstoffatom,
$R_1$ eine gegebenenfalls durch ein Halogenatom, eine Trifluormethyl- oder Alkylgruppe substituierten heteroaromatischen 6-gliedrigen Ring, der 1 oder 2 Stickstoffatome enthält,
$R_2$ ein Wasserstoffatom oder eine Hydroxygruppe,
$R_3$ eine Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe mit 1 bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, eine Alkoxygruppe mit 2 bis 7 Kohlenstoffatomen, die endständig durch eine Hydroxy-, Alkoxy- oder Phenylalkoxygruppe substituiert ist, oder eine gegebenenfalls durch eine Alkylgruppe substituierte Ethenylengruppe, die endständig durch eine Carboxy-, Alkoxycarbonyl-, Aminocarbonyl-, Alkylaminocarbonyl-, Dialkylaminocarbonyl-, Pyrrolidino-, Piperidino- oder Hexamethyleniminogruppe substituiert ist, und
n die Zahl 0 oder 1,
wobei alle vorstehend erwähnten Alkyl- oder Alkoxygruppen jeweils 1 bis 3 Kohlenstoffatome enthalten können, bedeuten, deren optische Isomere, Diastereomere und deren Säureadditionssalze.

2. Morpholine und Morpholin-N-oxide der Formel I gemäß Anspruch 1, in der
n wie im Anspruch 1 eingangs definiert ist,
A eine gegebenenfalls durch eine Methylgruppe substituierte Ethylen- oder n-Propylengruppe,
X eine Bindung,
$R_1$ einen gegebenenfalls durch ein Chlor- oder Bromatom substituierten Pyridinylrest, einen Pyrazinyl-, Pyrimidinyl-oder Pyridazinylrest,
$R_2$ ein Wasserstoffatom oder eine Hydroxygruppe und
$R_3$ eine Carboxy-, Methoxycarbonyl-, Ethoxycarbonyl-, Aminocarbonyl-, Carboxymethoxy-, Methoxycarbonylmethoxy-, Ethoxycarbonylmethoxy-, Aminocarbonylmethoxy-, Methylaminocarbonylmethoxy-, 2-Hydroxy-ethoxy-, 2-Ethoxy-ethoxy-, 2-Phenethoxy-ethoxy-, 6-Hydroxy-n-hexoxy- oder 2-Carbomethoxy-1-methyl-ethenylgruppe bedeuten, deren optisch Isomere, deren Diastereomere und deren Säureadditionssalze.

3. Morpholine und Morpholin-N-oxide der Formel

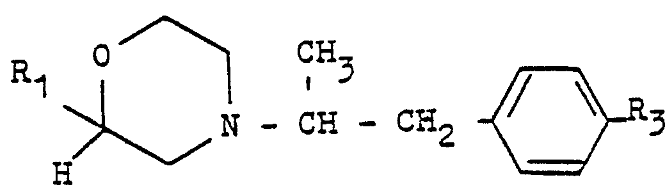

,(Ia)

in der
$R_1$ eine in 6-Stellung durch ein Chlor- oder Bromatom, durch eine Methyl- oder Trifluormethylgruppe substituierte Pyridin-2-ylgruppe und
$R_3$ eine Carboxymethoxy-, Carbomethoxymethoxy-, Ethoxycarbonylmethoxy-, Aminocarbonylmethoxy-, Methylaminocarbonylmethoxy-, 2-Hydroxy-ethoxy-, 2-Ethoxy-ethoxy- oder 2-Carbomethoxy-1-methyl-ethenylgruppe bedeuten,

deren optische Isomere, deren Diastereomere und deren Säureadditionssalze.

4. Als neue Verbindungen gemäß Anspruch 1:
N-[2-(4-Carbomethoxymethoxyphenyl)-1-methylethyl]-2-(6-chlor-pyridin-2-yl)-morpholin,
N-[2-(4-Carboxymethoxyphenyl)-1-methylethyl]-2-(6-chlor-pyridin-2-yl)-morpholin,
N-[2-(4-(2-Hydroxyethoxy)-phenyl)-1-methylethyl]-2-(6-chlor-pyridin-2-yl)-morpholin und
N-[2-(4-(2-Hydroxyethoxy)-phenyl)-1-methylethyl]-2-(6-chlor-pyridin-2-yl)-morpholin,
deren optische Isomere, deren Diastereomere und deren Säureadditionssalze.

5. N-[2-(4-(2-Hydroxyethoxy)-phenyl)-1-methylethyl]-2-(6-chlor-pyridin-2-yl)-morpholin, deren optische Isomere, deren Diastereomere und deren Säureadditionssalze.

6. Physiologisch verträgliche Salze der Verbindungen nach mindestens einem der Ansprüche 1 bis 5 mit anorganischen oder organischen Säuren.

7. Arzneimittel, enthaltend eine Verbindung nach mindestens einem der Ansprüche 1 bis 5 oder ein physiologisch verträgliches Säureadditionssalz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels, das für die Behandlung des Diabetes mellitus, der Adipositas und zur Behandlung und Prophylaxe atherosklerotischer Veränderungen geeignet ist.

9. Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

10. Verwendung einer Verbindung nach mindestens einem der Ansprüche 1 bis 6 als leistungsförderndes Mittel bei Tieren.

11. Tiernahrung und Prämixe zur Herstellung von Tiernahrung, enthaltend mindestens eine Verbindung nach mindestens einem der Ansprüche 1 bis 6.

12. Leistungsförderndes Mittel für Tiere, enthaltend mindestens eine Verbindung nach mindestens einem der Ansprüche 1 bis 6.

13. Verfahren zur Herstellung von leistungsfördernden Mitteln für Tiere, dadurch gekennzeichnet, daß eine Verbindung nach mindestens einem der Ansprüche 1 bis 6 mit Streck-, Verdünnungs- und Nahrungsmitteln sowie gegebenenfalls weiteren Hilfsstoffen vermischt wird.

14. Verfahren zur Herstellung der neuen Verbindungen nach mindestens einem der Ansprüche 1 bis 6, dadurch gekennzeichnet, daß

a) zur Herstellung von Verbindungen der Formel I, in der n die Zahl 0 darstellt, ein Keton der Formel

$R_1 - CO - CH_2 - Z_1$ ,(II)

in der
$R_1$ wie in mindestens einem der Ansprüche 1 bis 5 definiert ist und
$Z_1$ eine nukleophile Austrittsgruppe darstellt, mit einem Ethanolamin der Formel

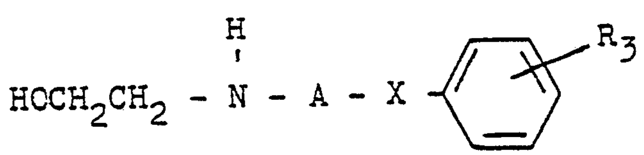
,(III)

in der
$R_3$, A und X wie in mindestens einem der Ansprüche 1 bis 5 definiert sind, umgesetzt und zur Herstellung einer Verbindung der Formel I, in der $R_2$ ein Wasserstoffatom darstellt, eine so erhaltene Verbindung der Formel

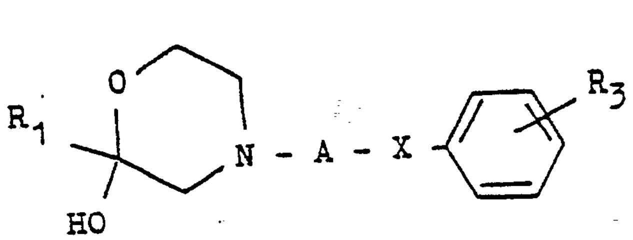
,(IV)

in der

$R_1$, $R_3$, A und X wie in mindestens einem der Ansprüche 1 bis 5 definiert sind, reduziert wird und gegebenenfalls anschließend eine so erhaltener Ester hydrolysiert wird oder

b) zur Herstellung von Verbindungen der Formel I, in der n die Zahl 0 und $R_2$ ein Wasserstoffatom darstellen, eine Carbonylverbindung der Formel

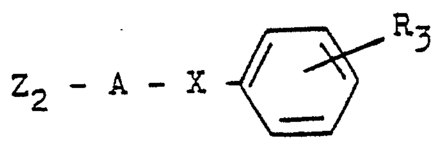

,(V)

in der

$R_3$, A und X wie in mindestens einem der Ansprüche 1 bis 5 definiert sind und

$Z_2$ zusammen mit einem Wasserstoffatom des benachbarten Kohlenstoffatoms des Restes A ein Sauerstoffatom darstellt mit einem Amin der Formel

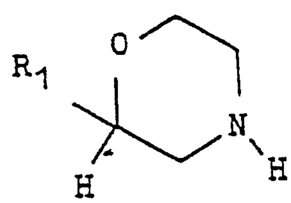

,(VI)

in der

$R_1$ wie in mindestens einem der Ansprüche 1 bis 5 definiert ist, reduktiv aminiert wird oder

c) zur Herstellung von Verbindungen der allgemeinen Formel I, in der n die Zahl 0 und $R_2$ ein Wasserstoffatom bedeuten, eine Verbindung der allgemeinen Formel

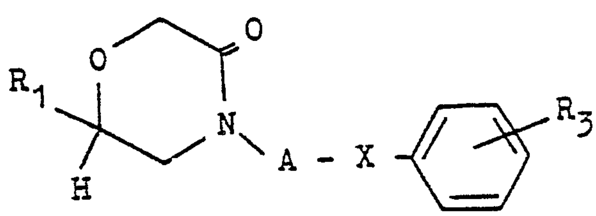

,(VII)

in der

A, X, $R_1$ und $R_3$ wie in mindestens einem der Ansprüche 1 bis 5 definiert sind, reduziert wird oder

d) zur Herstellung von Verbindungen der Formel I, in der n die Zahl 0, $R_2$ ein Wasserstoffatom und $R_3$ eine Alkoxygruppe mit 1 bis 3 Kohlenstoffatomen, eine Alkoxygruppe mit 1 bis 6 Kohlenstoffatomen, die endständig durch eine Carboxy-, Alkoxycarbonyl-, Aminocarbonyl-, Alkylaminocarbonyl-oder Dialkylaminocarbonylgruppe substituiert ist, oder eine Alkoxygruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, darstellen, eine Verbindung der Formel

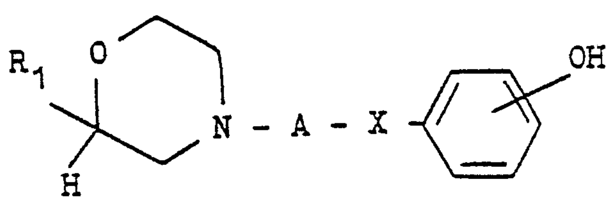

,(VIII)

in der

$R_1$, A und X wie in mindestens einem der Ansprüche 1 bis 5 definiert sind, mit einer Verbindung der Formel

$Z_3 - R_4$ ,(IX)

in der

$R_4$ eine Alkylgruppe mit 1 bis 3 Kohlenstoffatomen, eine Alkylgruppe mit 1 bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkylgruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, und

$Z_3$ eine nukleophile Austrittsgruppe wie ein Halogenatom oder eine Sulfonyloxygruppe oder

$Z_3$ zusammen mit einem $\beta$-Wasserstoffatom des Restes $R_4$ ein Sauerstoffatom darstellen, umgesetzt wird oder

e) zur Herstellung von Verbindungen der Formel I, in der n die Zahl 0 darstellt und $R_3$ eine Alkoxycarbonyl-, Aminocarbonyl-, Alkylaminocarbonyl- oder Dialkylaminocarbonylgruppe darstellt oder enthält, eine Verbindung der Formel

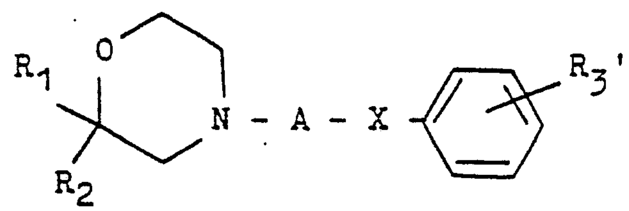
,(X)

in der

$R_1$, $R_2$, A und X wie in mindestens einem der Ansprüche 1 bis 5 definiert sind und

$R_3'$ eine Carboxygruppe oder eine Alkoxygruppe mit 1 bis 6 Kohlenstoffatomen, welche endständig durch eine Carboxygrup pe substituiert ist, darstellt, oder deren reaktionsfähige Derivate wie beispielsweise deren Ester, mit einer Verbindung der Formel

$H - R_5$ ,(XI)

in der

$R_5$ eine Alkoxy-, Amino-, Alkylamino- oder Dialkylaminogruppe darstellt, wobei jeweils der Alkyl- oder Alkoxyteil 1 bis 3 Kohlenstoffatome enthalten kann, umgesetzt wird oder

f) zur Herstellung der Verbindungen der Formel I, in der n die Zahl 1 darstellt, eine Verbindung der Formel

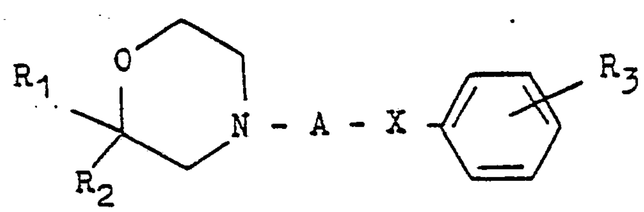
,(XII)

in der

$R_1$ bis $R_3$, A und X wie in mindestens einem der Ansprüche 1 bis 5 definiert sind, mit einer Peroxyverbindung oxidiert und gegebenenfalls eine so erhaltene Verbindung hydrolyisiert wird,

wobei bei den Umsetzungen a) bis f) reaktionsfähige Gruppen während der Umsetzung durch übliche Schutzreste geschützt sein können, welche nach der Umsetzung durch übliche Verfahren wieder abgespalten werden,

und gewünschtenfalls anschließend eine so erhaltene Verbindung der Formel I, in der $R_3$ eine Alkoxycarbonyl-, Aminocarbonyl-, Alkylaminocarbonyl- oder Dialkylaminocarbonylgruppe darstellt oder enthält, mittels Hydrolyse in eine entsprechende Verbindung der Formel I, in der $R_3$ eine Carboxygruppe darstellt oder enthält, übergeführt wird und/oder

eine so erhaltene Verbindung der Formel I, in der $R_3$ eine durch eine Carboxy-, Alkoxycarbonyl-, Aminocarbonyl-, Alkylaminocarbonyl- oder Dialkylaminocarbonylgruppe substituierte Alkoxygruppe darstellt, mittels Reduktion durch ein geeignetes Metallhydrid in eine Verbindung der Formel I, in der der vorstehend erwähnte substituierte Alkoxyrest anstelle der Carbonylgruppe eine Methylengruppe enthält, übergeführt wird und/oder

eine so erhaltene Verbindung der Formel I, in der $R_3$ eine der in den Ansprüchen 1 bis 5 erwähnten Alkoxygruppen darstellt, mittels Etherspaltung in eine entsprechende Verbindung der Formel I, in der $R_3$

eine Hydroxygruppe oder eine durch eine Hydroxygruppe substituierte Alkoxygruppe darstellt, übergeführt wird und/oder
eine so erhaltene Verbindung der Formel I, sofern diese ein oder mehrere optische aktive Kohlenstoffatome enthält, in ihre optischen Isomere und Diastereomere aufgetrennt wird und/oder
eine so erhaltene Verbindung der Formel I in ihre Säureadditionssalze, insbesondere in ihre physiologisch verträglichen Säureadditionssalze übergeführt werden.

Europäisches Patentamt

# EUROPÄISCHER RECHERCHENBERICHT

Nummer der Anmeldung

EP 89 10 4376

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 140 359 (BEECHAM)<br>* Ansprüche *<br>--- | 1,7-14 | C 07 D 413/04<br>A 61 K 31/535//<br>C 07 D 213/61 |
| A | DE-A-2 121 031 (KARL THOMAE)<br>* Ansprüche *<br>----- | 1,7-14 | |
| | | | **RECHERCHIERTE SACHGEBIETE (Int. Cl.4)** |
| | | | C 07 D 413/00<br>C 07 D 265/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 23-06-1989 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)